# EUROPEAN PATENT SPECIFICATION

(11) **EP 4 640 268 B1**
(45) Date of publication and mention of the grant of the patent: **14.10.2026**
(21) Application number: 24172356.8
(22) Date of filing: 25.04.2024
(51) Int. Cl.: A61N 1/05, A61N 1/375

(54) **BIOMEDICAL APPARATUS FOR IMPLANTATION WITHIN LIVING BODIES**
BIOMEDIZINISCHES GERÄT ZUR IMPLANTATION IN LEBENDE KÖRPER
APPAREIL BIOMÉDICAL POUR IMPLANTATION DANS DES CORPS VIVANTS

(43) Date of publication of application: 29.10.2025
(73) Proprietor: Mehta, Mitul, 2930 Brasschaat (BE)
(72) Inventor: Mehta, Mitul, 2930 Brasschaat (BE)
(74) Representative: Renaudo, Adrien Hanouar

(56) References cited:
- US-A1- 2002 021 784
- US-A1- 2012 232 371
- US-A1- 2020 303 236
- SAMANTHA G. LICHTER ET AL: "Hermetic diamond capsules for biomedical implants enabled by gold active braze alloys", BIOMATERIALS, vol. 53, 1 June 2015 (2015-06-01), AMSTERDAM, NL, pages 464 - 474, XP055533478, ISSN: 0142-9612, DOI: 10.1016/j.biomaterials.2015.02.103

## Description

### Technical Field

The present disclosure relates to a biomedical apparatus for implantation within a living body. More particularly, the present disclosure relates to an implantable biomedical apparatus including an engineered diamond casing.

### Background

Electronic systems and apparatuses, such as implantable biomedical apparatuses, are generally configured to be partially or entirely inserted into a living body for assisting with the execution of one or more bodily functions. The implantable device may be used in a wide range of applications including pacemakers, defibrillators, neurostimulators, drug delivery systems, biosensors, and visual/auditory prostheses. An implantable biomedical apparatus commonly includes one or more electronic components, such as an integrated circuit board, e.g., for providing electrostimulation to a part of the living body and/or one or more sensors for sensing parameters within the living body. During an operation of such an electronic component, one region of the electronic component can dissipate more heat than another region of the electronic component. Ineffective heat dissipation may lead to localized build-up of heat or hot spots around the associated system. Such excessive heat or hot spots can damage surrounding living tissue and/or cells and may also reduce an overall working life of the electronic component. Further, for an implantable device used in visual/auditory prostheses, excessive heat may lead to light wavelength shifts, light beam divergence, thereby reducing optical or acoustic efficiency of the device. Similarly, for pacemakers and defibrillators, ineffective heat distribution may affect the timing accuracy and stability of electrical impulses, potentially causing irregularities in the cardiac stimulation.

United States Patent No.: 10,434,235 discloses an implantable wireless power receiver including at least one thermal layer disposed on an interior surface of the receiver configured to conduct heat from a central portion of the receiver towards edges of the receiver. The thermal layer may comprise, for example, a copper layer or a ceramic layer embedded in an acrylic polymer matrix. In some embodiments, a plurality of thermal channels can be formed within the receiver to transport heat from the central regions of the receiver towards the edges of the receiver via free convection. US patent application publication US 2012232371A1 discloses a method for producing a biomedical apparatus for implantation within a living body, the biomedical apparatus comprising: an electrode; an electronic device configured to transmit electrical signals to a portion of the living body through the electrode; and a monolithic diamond casing for housing and hermetically sealing the electronic device and the electrode therewithin, wherein the monolithic diamond casing includes: a first portion defining an encapsulating layer around the electrode, the first portion configured to define an electrical path between the electrode and the portion of the living body; and a second portion enwrapping the electronic device and the electrode and being integral and seamlessly contiguous to the first portion.

### Summary

The present invention is defined by the appended claims. Further aspects, embodiments and examples described hereinafter are only of exemplary nature and are presented for better understanding the present invention.

In one aspect, the present disclosure relates to a biomedical apparatus for implantation within a living body. The biomedical apparatus includes an electrode and an electronic device configured to transmit electrical signals to a portion of the living body through the electrode. The biomedical apparatus further includes a monolithic diamond casing for housing and hermetically sealing the electronic device therewithin. The monolithic diamond casing includes a first portion abutting and defining an encapsulating layer around the electrode. The first portion is configured to define an electrical path between the electrode and the portion of the living body. The monolithic diamond casing further includes a second portion enwrapping the electronic device and being integral and seamlessly contiguous to the first portion, the second portion defining one or more heat dissipation structures to dissipate heat generated from the electronic device away from the monolithic diamond casing.

In another aspect, the present disclosure relates to a method for producing a biomedical apparatus. The method comprises providing a first support structure including a first seed layer of diamond inside a chemical vapor deposition (CVD) reactor. The first support structure is one of a mold or a template. The method further includes forming a first layer of diamond on the first seed layer to form a first portion of a monolithic diamond casing. The method further includes debonding the first portion of the monolithic diamond casing from the first support structure. The method further includes providing a second support structure including a second seed layer of diamond inside the CVD reactor, wherein the second support structure is one of a mold or a template. The method includes forming a second layer of diamond on the second seed layer to form a second portion of a monolithic diamond casing. The method includes debonding the second portion of the monolithic diamond casing from the second support structure. The method further includes positioning an electrode on the first portion such that the first portion abuts and defines an encapsulating layer around one or more tips of the electrode, the first portion configured to define an electrical path between the electrode and a portion of a living body. The method further includes providing an electronic device on the first portion to transmit electrical signals to a portion of the living body through the electrode. The method further includes joining the first portion and the second portion to form the monolithic diamond casing such that the second portion enwraps the electronic device and is integral and seamlessly contiguous to the first portion. The second portion defines one or more heat dissipation structures to dissipate heat generated from the electronic device away from the monolithic diamond casing.

### Brief Description of the Drawings

FIG. 1 is a perspective view of a biomedical apparatus, in accordance with some embodiments of the present disclosure;
FIG. 2 is a side view of the biomedical apparatus of FIG. 1, in accordance with some embodiments of the present disclosure;
FIG. 3 is a cross-sectional view of the biomedical apparatus of FIG. 1 along a line 3-3, in accordance with some embodiments of the present disclosure;
FIG. 4 is a cross-sectional view of the biomedical apparatus of FIG. 1 along a line 4-4, in accordance with some embodiments of the present disclosure;
FIG. 5 is a cross-sectional view of the biomedical apparatus of FIG. 4 illustrating certain internal details of the biomedical apparatus, in accordance with some embodiments of the present disclosure;
FIG. 6 is perspective bottom view of the biomedical apparatus of FIG. 1, in accordance with some embodiments of the present disclosure;
FIGS. 7A-7D are perspective views of the biomedical apparatus of FIG. 1, including a diamond casing having different shapes, in accordance with some embodiments of the present disclosure;
FIGS. 8A-8F are method steps of producing the biomedical apparatus of FIG. 1, in accordance with some embodiments of the present disclosure; and
FIGS. 9 is a method for producing the biomedical apparatus of FIG. 1, in accordance with some embodiments of the present disclosure.

### Detailed Description

Reference will now be made in detail to specific embodiments or features, examples of which are illustrated in the accompanying drawings. Wherever possible, the same reference numbers will be used throughout the drawings to refer to the same or the like parts.

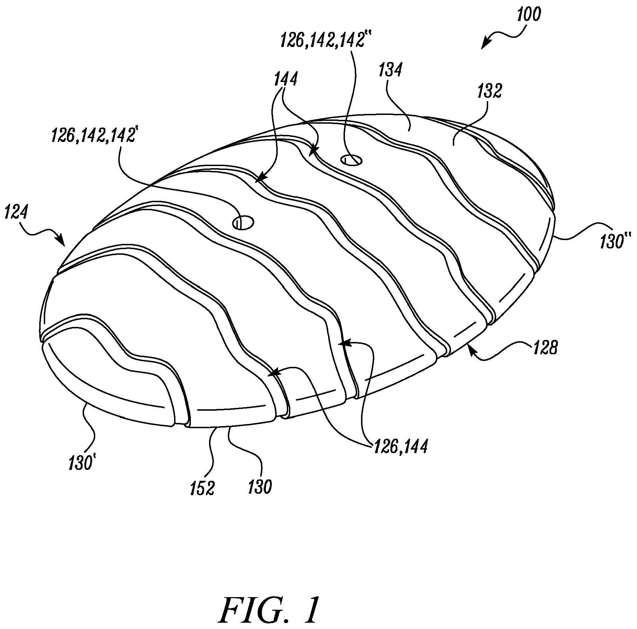

Referring to FIG. 1, an exemplary biomedical apparatus 100 is disclosed. The biomedical apparatus 100 may be configured to be implanted within a living body. As an example, the biomedical apparatus 100 may be configured to be surgically implanted within a living body to perform one or more of: diagnose, monitor, manage, or treat a wide range of medical conditions in a patient's body, e.g., in a living body or in living being, although aspects of the present disclosure may be applied in living beings which are not living beings. The biomedical apparatus 100 may be used in regulating heart rhythms, managing chronic pain, restoring sensory functions, controlling metabolic disorders in the living body, and the like medical conditions. The biomedical apparatus 100 is engineered to be biocompatible with the living body. "Biocompatible" may refer to a material or a substance of the biomedical apparatus 100 that is compatible with living tissues or organisms, meaning it does not produce harmful or toxic effects when it comes into contact with biological systems. Moreover, these materials are designed to interact with living tissues in an intended way that is safe and non-reactive, promoting healing and integration with surrounding tissues of the living body.

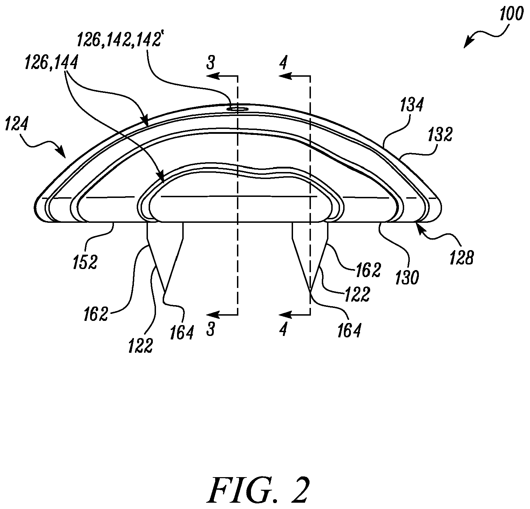

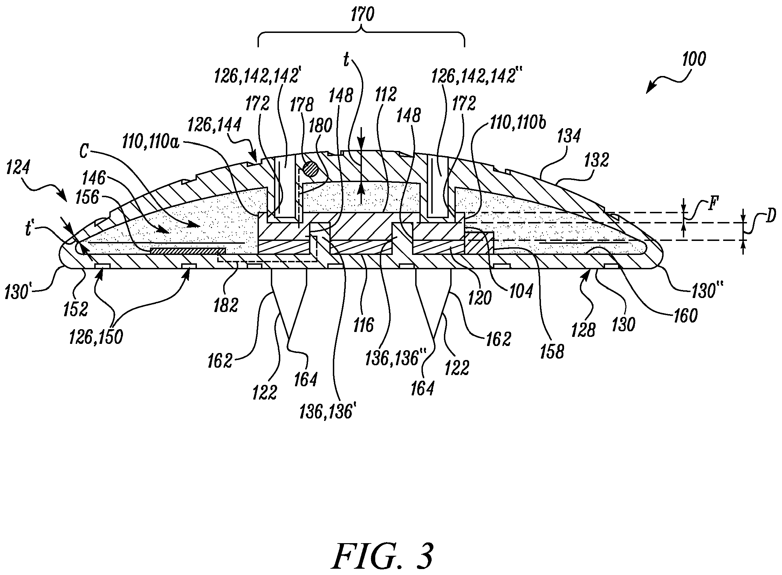

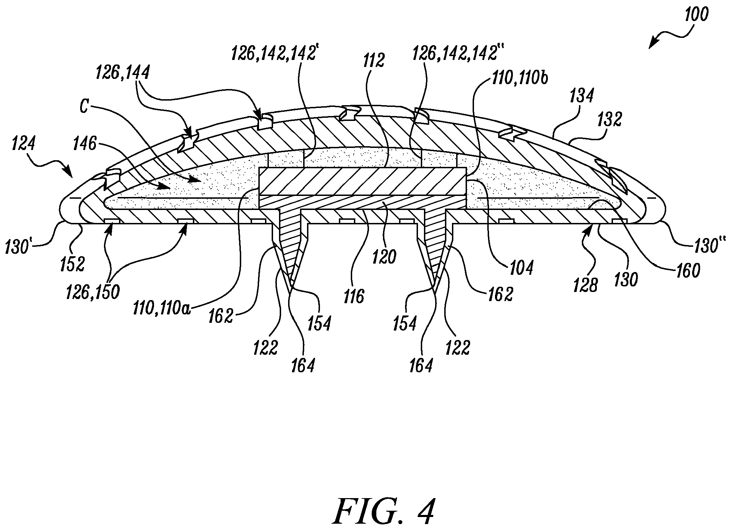

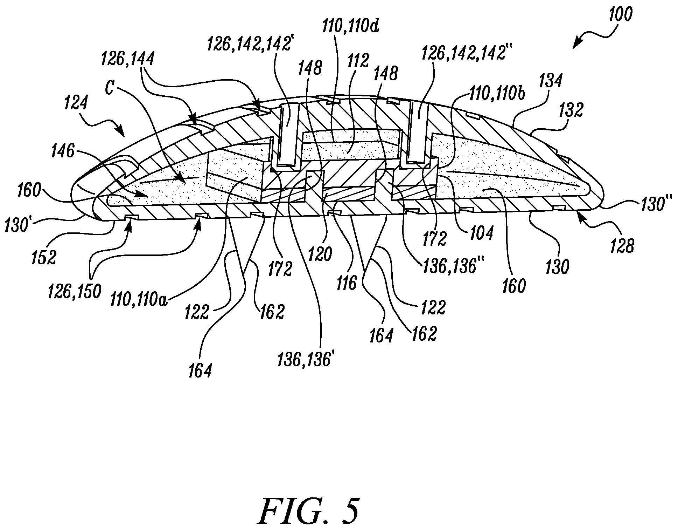

Referring to FIG. 2 through 6, the biomedical apparatus 100 includes an electronic device 104. The electronic device 104 may define an upper surface or a first side 112 and a lower surface or a second side 116. The electronic device 104 may also define a series of lateral faces 110, e.g., a first lateral face 110a, a second lateral face 110b, a third lateral face 110c, and a fourth lateral face 110d, each extending from the first side 112 to the second side 116. The first side 112 may be disposed oppositely to the second side 116. Further, the first lateral face 110a may be located oppositely to the second lateral face 110b, and the third lateral face 110c may be located oppositely to the fourth lateral face 110d. The electronic device 104 may include one or more first slots 148 (e.g., see slot 148' and slot 148") formed on any part of the second side 116 of the electronic device 104. The position of the slots 148 on electronic device 104, as illustrated in any one or more of the accompanying figures, is exemplary and the slots 148 can be located elsewhere on the electronic device 104. In some embodiments, the slots 148 may be formed in a region of the electronic device 104 where the chances of developing the hotspots are relatively more as compared to other regions of the electronic device 104 where the chances of developing the hotspots are relatively less. Further, in some embodiments, the slots 148 formed on the electronic device 104 may be formed up to a depth, D, into the electronic device 104 (see FIG. 3). The electronic device 104 may further include one or more second slots 172 formed on any part of the first side 112 of the electronic device 104. The position of the slots 172 on the electronic device 104, as illustrated in any one or more of the accompanying figures, is exemplary and the slots 172 can be located elsewhere on the electronic device 104. In some embodiments, the slots 148 may be formed in a region of the electronic device 104 where the chances of developing the hotspots are relatively more as compared to other regions of the electronic device 104 where the chances of developing the hotspots are relatively less. Further, in some embodiments, the slots 172 formed on the electronic device 104 may be formed up to a depth, *F*, into the electronic device 104 (see FIG. 3).

The electronic device 104 further includes an electrode 120. The electrode 120 is configured to transmit electrical signals to a portion of a living body. As an example, the electronic device 104 may be configured to provide electrical signals to a portion of the living body through the electrode 120 for electrically stimulating the portion of the living body. In some embodiments, the portion of the living body may be a living tissue within the living body. The electrode 120 may be connected to the second side 116 of the electronic device 104 and may be connected to a battery 158 for providing power to the electrode 120. In some embodiments, the electrode 120 may include one or more sensors for sensing signals corresponding to a physiological state within the living body. In some embodiments, the physiological state may include a function and/or a condition of the living body. In some embodiments, the electrode 120 may each define one or more tips 154 (e.g., two tips) located away from the electronic device 104. In some embodiments, the tips 154 may be sharp tips. In some embodiments, the electrode 120 may include provisions for attaching one or more wires to enable electrical stimulation or sensing of the portion of the living body that is distant from the portion of the living body where the biomedical apparatus 100 is placed. The wires may be made of biocompatible conductive materials such as, but not limited to, platinum, iridium, or stainless steel, and insulated with materials such as, but not limited to, silicone or Parylene C. The wires may be routed from the diamond casing 124 through one or more hermetically sealed holes to the portion of the living body. This allows the biomedical apparatus 100 to provide therapy or diagnostics to one or more regions which are away from the biomedical apparatus 100. The wires may be connected to additional electrodes or sensors at the distal end placed away from the biomedical apparatus 100. For example, the wires may terminate in a nerve cuff, intramuscular electrode, or other stimulating or sensing element suited to a specific medical application.

In some embodiments, the electronic device 104 may include various components, such as an antenna 156 and/or the battery 158 (see FIG. 3). The battery may be used for supplying power to the electrode 120 and the antenna 156 may be used for transmitting and receiving signals (e.g., radio frequency signals) from the electronic device 104 to an external device placed outside the living body. In some embodiments, the antenna may be part of a transceiver of the electronic device 104. In some embodiments, the antenna 156 may be connected to the electronic device 104 through one or more conductive paths embedded within the diamond casing 124. For example, the antenna 178 may be connected to the electronic device 104 by using one or more metal wires 182 made of material such as gold or platinum, embedded within the diamond casing 124 (see FIG. 3). In some embodiments, the antenna 156 and/or the battery 158 may be disposed on the electronic device 104. In some embodiments, the electronic device 104 may include one or more electronic units stacked together. For example, the electronic device 104 may include one stimulation unit stacked onto one sensing unit. In some embodiments, the battery 158 may also be stacked with such electronic units.

The biomedical apparatus 100 further includes a monolithic diamond casing 124. The monolithic diamond casing 124 is configured for housing and hermetically sealing the electronic device 104 therewithin. The diamond casing 124 includes a first portion 128 and a second portion 132.

The first portion 128 may correspond to a base 130 of the monolithic diamond casing 124 and the same may be used for coupling and mounting the electronic device 104 and the electrode 120 thereto, i.e., into the monolithic diamond casing 124. The first portion 128 may provide a surface (e.g., see inner side 160) for mounting the second side 116. The first portion 128 may be configured to abut and define an encapsulating layer 122 around the electrode 120 to define an electrical path between the electrode 120 and the portion of the living body. In some embodiments, the first portion 128 and/or the encapsulating layer 122 defines receptacles 162 to receive the tips 154 of the electrode 120. The receptacles 162 themselves may define ultrafine tips 164 to complementarily receive the tips 154 (e.g., the sharp tips of the electrode therewithin). In some embodiments, the ultrafine tips 164 may have diameters ranging from 0.1 µm to 5 µm and lengths of 10 µm to 100 µm., depending on the specific application and target tissue of the biomedical apparatus 100. For example, the encapsulating layer 122 and/or the receptacles 162 formed around the electrode 120 may include a tapered profile across at least part of its expanse to form ultrafine tips 164. The ultrafine tips 164 may easily be connected to a living tissue within the living body. In some embodiments, the ultrafine tips 164 may be inserted into a nerve tissue within the living body.

The dimensions of the ultrafine tips 164 may be chosen to optimize a balance between selectivity, sensitivity, and stability of the electrode-tissue interface, while minimizing tissue damage and ensuring reliable electrical contact. For example, smaller tip dimensions may be suitable for applications requiring high spatial precision and targeting of individual cells or small populations of cells, while larger tip dimensions may be suitable for applications requiring robust and stable electrical coupling with larger tissue volumes. Effectively, dimensions of the ultrafine tips 164 can be tailored to accommodate one or more unique requirements of a biomedical application, such as neural recording and stimulation, cardiac pacing and sensing, and sensory prostheses.

In some embodiments, the encapsulating layer 122 may include one or more sensors disposed therein. In some embodiments, the electrode 120 is an integral part of the monolithic diamond casing 124. In such a scenario, the electrode 120 is formed from the same material as that of the diamond casing 124. For example, the material of the electrode 120 is same as the encapsulating layer 122 of the diamond casing 124.

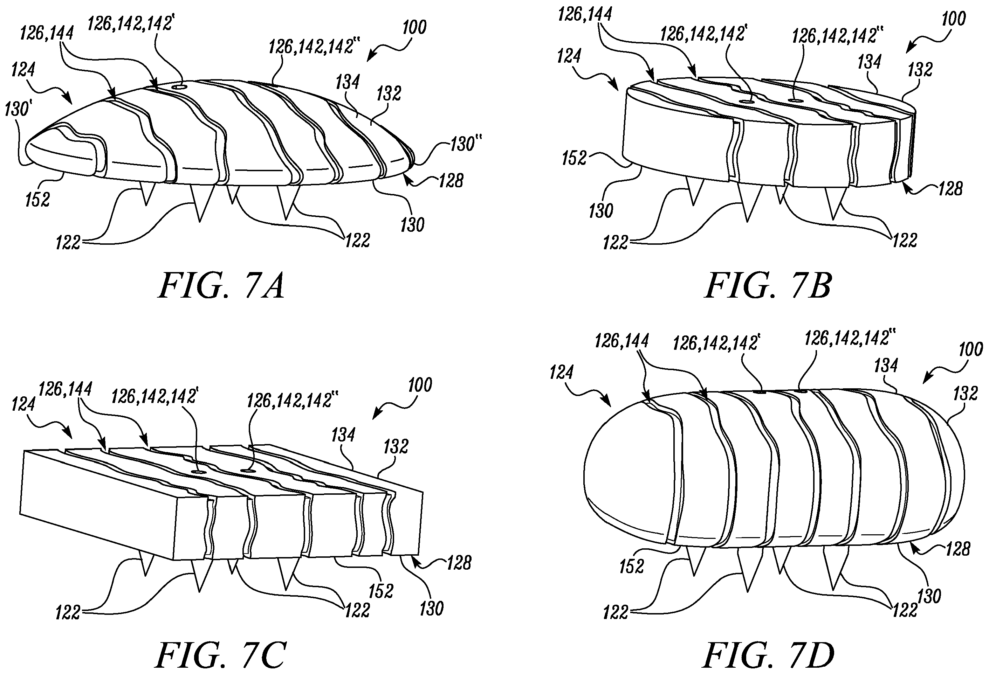

In some embodiments, the first portion 128 includes a circular shape. Other shapes and designs, such as an ovular shape, an oblong shape, a rectangular shape, etc., may be contemplated. In some embodiments, the base 130 may include a constant cross-section across its expanse, e.g., from one diametric end 130' of the base 130 to another diametric end 130" (e.g., along a major axis) of the base 130, as shown. Although the base 130 may include a varying cross-section depending on the heat dissipating requirements of the electronic device 104. For example, the cross-section area of the base 130 may be relatively more in certain regions, e.g., which may enable excess heat to be absorbed or extracted thereto, e.g., from hotspots defined by the electronic device 104, or from locations of the electronic device 104 which may produce relatively excessive heat. Although not limited, the diamond casing 124, as a whole, may include a spherical or an oval shape (see FIG. 7A). Other shapes and designs may be contemplated. In some embodiments, the diamond casing 124 may include a cylindrical shape (see FIG. 7B), a rectangular shape (see FIG. 7C), a capsular shape (see FIG. 7D), and the like. Therefore, the shape of the diamond casing 124 as illustrated in the FIGS. 1 through 6 is provided for illustrative purposes only. Those skilled in the art may select a shape of the diamond casing 124 from one or more shapes of the diamond casing 124 described in the present disclosure, or may contemplate various other shapes without departure from the claimed subject matter.

In some embodiments, the first portion 128 further includes one or more first thermal vias 136 (e.g., see first thermal vias 136' and 136") (see FIG. 3). The thermal vias 136 may extend from the base 130, and may ingress, at least partly, into slots 148 formed on the electronic device 104 for transferring heat from the electronic device 104 to the base 130, although in some cases, the thermal vias 136 may just abut with the electronic device 104. By way of the abutment or the ingression, the thermal vias 136 may be configured for providing a transfer route to a heat generated by the electronic device 104 to the base 130. In some embodiments, the thermal vias 136 may be ingressed at corresponding regions of the electronic device 104 where the chances of developing the hotspots are relatively more than other of its regions where the chances of developing the hotspots are relatively less. In some embodiments, the thermal vias 136 may include a varying cross-sectional area across its expanse.

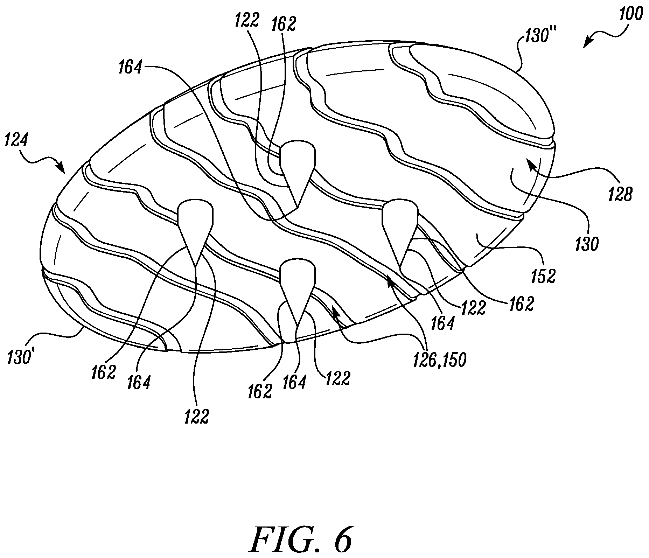

In some embodiments, the first portion 128 includes one or more heat dissipation structures 126 to dissipate heat generated from the electronic device 104 away from the monolithic diamond casing 124. In some embodiments, the heat dissipation structures 126 may include microchannels 150. The microchannels 150 may include one or more irregular, asymmetrical and/or nonlinear shaped or profiled microchannels 150 (see FIG. 6). As further examples, the microchannels 150 may be formed in fractal profile, sinusoidal profile, grid profile, or any combination thereof. The microchannels 150 may be provided on an outer side 152 of the first portion 128. Also, the microchannels 150 may be engraved into the first portion 128 (e.g., the microchannels 150 may extend into a body of the first portion 128 from the outer side 152) (see FIG. 6).

The second portion 132 is configured to enwrap the electronic device 104. The second portion 132 is integral and seamlessly contiguous to the first portion 128. The second portion 132 outwardly projects from the first portion 128. The second portion 132 includes a dome 134 disposed on the base 130 and may be integrally formed with the base 130. The dome 134 may at least partly surround the electronic device 104. Further, the dome 134 defines a clearance, C, within the monolithic diamond casing 124 with respect to the base 130 and the electronic device 104. Therefore, although not limited, owing to the clearance, C, the dome 134 may not abut with the base 130 and the electronic device 104 and may be spaced apart from the base 130 and the electronic device 104. The dome 134 includes an outer side 138 and an inner side 140. In some embodiments, the clearance C is variable between the diametric ends 130', 130", with the clearance, C, being maximum at a portion (e.g., a central portion 170) where the electronic device 104 is mounted to the base 130. In some embodiments, the dome 134 may include one or more sensors 178 disposed therein (see FIG. 3). The sensor 178 is embedded within the dome 134 thereby allowing the sensor 178 to be in direct contact with the portion of the living body. In some embodiments, a signal frequency of the one or more sensors 178 and/or the antenna 156 may be different to avoid interference of the signals from the sensors 178 and/or the antenna 156. In some embodiments, the electronic device may employ different frequency separation techniques such as, but not limited to, bandpass filtering, time-division multiplexing, spatial separation, and the like to avoid interference of the signals from the sensors 178 and/or the antenna 156 thereby enabling the sensors 178 and the antenna 156 to operate simultaneously without compromising signal integrity or signal quality.

Further, the second portion 132 includes one or more heat dissipation structures 126 to dissipate heat generated from the electronic device 104 away from the monolithic diamond casing 124. In some embodiments, the heat dissipation structures 126 may include one or more second thermal vias 142 (e.g., see second thermal vias 142' and 142") extending from the dome 134 and being in contact with, or ingressing, at least party, into electronic device 104. In some embodiments, the heat dissipation structures 126 may include one or more irregular, asymmetrical and/or nonlinear microchannels 144. For example, the microchannels 144 may be formed in fractal profile, sinusoidal profile, grid profile, or any combination thereof. The microchannels 144 may be provided on an outer side 138 of the dome 134. Also, the microchannels 144 may be engraved into the dome 134 (e.g., the microchannels 144 may extend into a body of the dome 134 from the outer side 138). In some embodiments, the microchannels 144 may include an irregular cross-section partly or throughout/across their expanse. In some embodiments, the microchannels 144 may have varying depth and width. In some embodiments, the microchannels 144 may be different from each other. In some embodiments, the microchannels 144 formed on the second portion 132 may be similar to or different from the microchannels 150 formed on the first portion 128.

The aforesaid geometry of the microchannels 144, 150 allows thermal management for electronic device 104 and maintaining the temperature of the electronic device 104, in turn enabling the electronic device 104 to function in an optimal condition. For example, the aforesaid geometry of the microchannels 144 and 150 results in an increase in an outer surface area of the diamond casing 124, thereby allowing relatively more heat dissipation from the electronic device 104 in turn keeping the electronic device 104 in an optimal working temperature. In some embodiments, the microchannels 144, 150 may be filled with a coolant or a phase-change material configured to further enhance the heat dissipation capabilities. In some embodiments, the coolant or the phase-change material may be biocompatible and non-toxic. In some embodiments, the diamond casing 124 may incorporate sealing techniques, such as, but not limited to hermetic bonding or atomic layer deposition (ALD) of protective coatings, to create an impenetrable barrier between the microchannels 144, 150 and the surrounding environment. The protective coatings ensure that the coolant or the phase-change material remains securely contained within the microchannels 144, 150 thereby eliminating any risk of leakage and maintaining the safety and integrity of the biomedical apparatus 100 within the living body.

In some embodiments, the second portion 132 may include a varying cross-sectional area across its expanse, depending on the heat dissipating requirements of the electronic device 104. For example, heat dissipating requirements may include, but not limited to, a rate of removal of heat from the electronic device 104, an amount of heat required to be lowered from the electronic device 104 for optimal operation of the electronic device 104. For example, the thickness of the dome 134 may be relatively more in certain regions to enable excess heat to be absorbed or extracted thereto, e.g., from hotspots defined by the electronic device 104 or from locations of the electronic device 104 which may produce relatively excessive heat. For example, a thickness (t) of the central portion 170 of the dome 134 is more than the thickness (t') of a side portion of the dome 134 (see FIG. 3). For example, the regions of the second portion 132 having greater cross-sectional area dissipate relatively more heat than the regions of the second portion having lower cross-sectional area.

In some embodiments, the second portion 132 has a thickness ranging from 0.7 mm to 10 mm. Further, a thermal conductivity of the second portion 132 may lie between 500 to 2500 W/mK at 25°C. In some examples, the second portion 132 may be configured to handle a heat flux that lies between 100 to 1000W/cm² (watts per centimeter squared). The above examples and numerals are provided for illustrative purposes, and may include other values. In some embodiments, a thermal interface material 146 or microfluidics may be placed between the first portion 128 and the second portion 132, and may occupy the clearance, C. In some embodiments, the clearance, C, is a vacuum present between the first portion 128 and the second portion 132.

As a whole, the diamond casing 124 may include non-uniform or different grain size distributions and varying grain orientations configured for enhancing the thermal conductivity of the diamond casing 124. For example, the regions of the diamond casing 124 having a lower grain size distribution provide relatively more thermal conductivity than the regions of the diamond casing 124 having a higher grain size distribution. This is due to the reduction of phonon scattering at grain boundaries in smaller grains, which allows for more efficient heat transfer. In addition to grain size, the thermal conductivity of diamond is also influenced by factors such as the presence of defects, impurities, and isotopic composition. Generally, grain size and grain orientation may be controlled through process parameters, such as, deposition temperature, use of dopants, substrate selection, surface treatment, and controlled nucleation, and the like. In an exemplary embodiment, grain size distributions may be achieved by altering a ratio of atomic hydrogen to methane gas flow rate during a process of doping the hydrogen and methane.

In some embodiments, higher methane percentage in the diamond deposition process promotes the growth of existing diamond grains rather than the formation of new grains. As a result, large continuous diamond crystals with reduced grain boundary density are formed. The grain size and morphology can be further controlled by adjusting other process parameters, such as, but not limited to, a ratio of methane to hydrogen, a total gas pressure, the substrate temperature. In some embodiments, different grain orientations may be achieved by processes including, but not limited to, abrasive mechanical polishing, ion beam texturing, lithography, and the like. Additionally, substrate material and crystallographic orientation may influence the grain orientation of the deposited diamond. In some embodiments, the diamond may be a nanocrystalline diamond having a grain size of approximately 50-300 nm (nanometer). In some embodiments, the diamond may be a polycrystalline diamond having a grain size of approximately 200-300 nm.

As a whole, the monolithic diamond casing 124 may be produced from a single crystal or polycrystalline diamond. The single crystal and/or polycrystalline diamond may provide thermal conduction greater than 1500 W/mK (Watts per meter-Kelvin) at room temperature (e.g., 20 °C). In some embodiments, the diamond casing 124 may be manufactured by doping one or more gases including, but not limited to, boron nitrogen, or hydrogen to enhance the electrical and thermal conductivity of the diamond casing 124. The diamond casing 124 may be manufactured by using a method exemplarily discussed below.

### Industrial Applicability

In some embodiments, the exemplary method of producing the diamond casing 124 may include utilizing a computational model to determine the heat dissipation requirements for the electronic device 104. For example, a computational model may be used to determine an amount of heat generated by the electronic device 104 and the regions of hotspot formation in the electronic device 104. Further, based on the amount of heat generated and the determined regions of hotspot formation in the electronic device 104, the method includes preparation of a diamond casing 124.

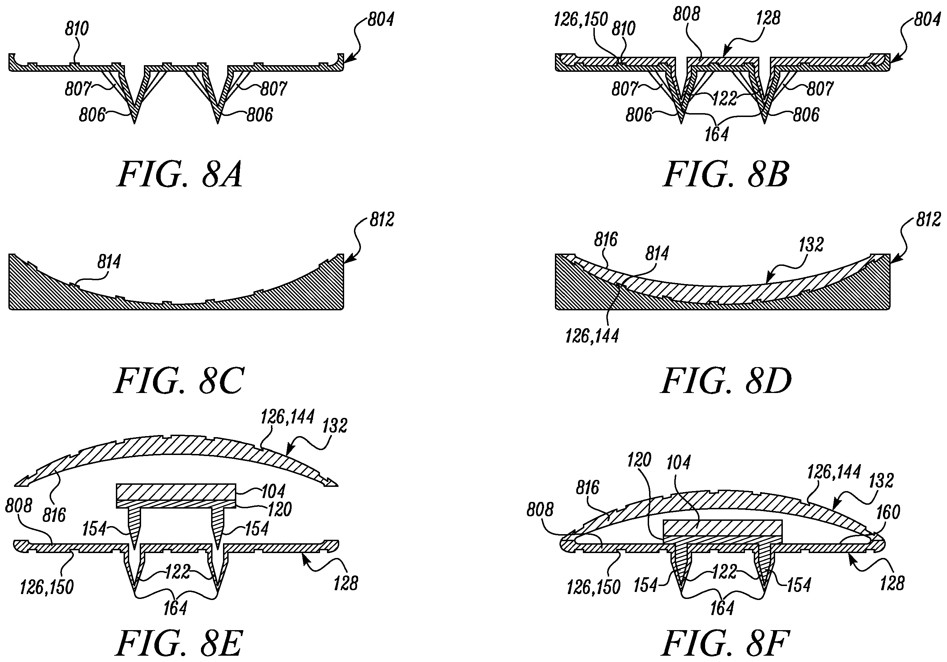

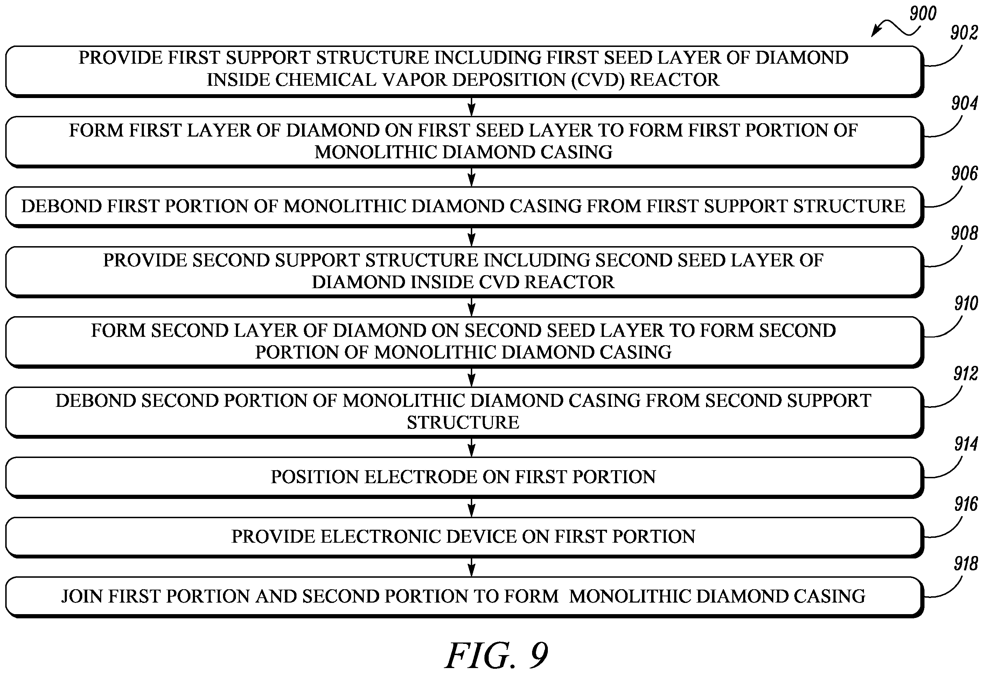

FIG. 9 describes an exemplary method 900 for producing the diamond casing 124. The method 900 is discussed by way of a flowchart and is discussed in conjunction with FIGS. 8A to 8F, as well. It will be appreciated that the order of steps described in the method 900 is exemplary in nature and that the steps can be performed in a different order than what is set out below, as will be contemplated by a person skilled in the art based on the description of the present disclosure.

The method 900 begins with providing a first support structure 804 including a first seed layer of diamond inside a chemical vapor deposition (CVD) reactor at block 902 (see FIG. 8A). The first support structure 804 may be one of a mold or a template. The first seed layer may be deposited on the first support structure 804. In some embodiments, the first seed layer may include a thin layer of diamond deposited on the support structure 804. In some embodiments, one or more of a nanodiamond or microdiamond may be deposited onto the support structure 804 to form the first seed layer. The support structure may be made of any suitable material, including but not limited to, semiconductor, metal, and insulator materials such as silicon, silicon dioxide, and the like. The first support structure 804 may include receptacles 806 for the formation of tips 154 of the electrode 120 (see FIG. 8A). In some embodiments, the receptacles 806 may be supported by one or more scaffolding 807 (as shown in FIG. 8A-8B). Further, the first support structure 804 may include protrusions 810 for formation of microchannels 144 (see FIG. 8A). Similarly, the first support structure 804 may include similar features to form one or more cavities, thermal vias, electronic slot, and the like. At block 904, the method includes forming a first layer 808 of diamond on the first seed layer to form the first portion 128 of the monolithic diamond casing 124 (see FIG. 8B). At block 906, the method includes debonding the first portion 128 of the monolithic diamond casing 124 from the first support structure 804.

At block 908, the method includes providing a second support structure 812 including a second seed layer of diamond inside the CVD reactor (see FIG. 8C). The second support structure 812 may be one of a mold or a template. In some embodiments, the second seed layer may include a thin layer of diamond deposited on the second support structure 812. In some embodiments, one or more of a nanodiamond or microdiamond may be deposited onto the second support structure 812 to form the second seed layer. The second support structure 812 may include protrusions 814 for the formation of microchannels 150 (see FIG. 8C). At block 910, the method includes forming a second layer 816 of diamond on the second seed layer to form the second portion 132 of a monolithic diamond casing 124 (see FIG. 8D). At block 912, the method includes debonding the second portion 132 of the monolithic diamond casing 124 from the second support structure 812. At block 914, the method includes positioning the electrode 120 on the first portion 128 such that the first portion 128 abuts and defines an encapsulating layer around one or more tips 154 of the electrode 120 (see FIG. 8E). At block 916, the method includes providing an electronic device 104 on the first portion 128 to transmit electrical signals to the portion of the living body through the electrode 120 (see FIG. 8E). In some embodiments, the electronic device 104 may be positioned and secured onto a predefined area of the first portion 128, ensuring proper alignment and orientation. At block 918, the method includes joining the first portion 128 and the second portion 132 to form the monolithic diamond casing 124 such that the second portion 132 enwraps the electronic device 104 and is integral and seamlessly contiguous to the first portion 128 (see FIG. 8F). The joining of the first portion 128 and the second portion 132 may be performed by any suitable method such as laser welding. The second portion 132 defines one or more heat dissipation structures 126 to dissipate heat generated from the electronic device 104 away from the monolithic diamond casing 124. After, the formation of the diamond casing 124, the method further includes one or more post-growth processing steps, such as, but not limited to surface polishing, laser micromachining, to provide a surface finish to the diamond casing 124, such that the second portion 132 enwraps the electronic device 104 and is integral and seamlessly contiguous to the first portion 128.

In some embodiments, the formation of the first layer 808 and the second layer 816 may include filling the CVD reactor with a mixture of gases, such as, but not limited to carbon-containing gas (e.g., methane and hydrogen). The gases may be ionized and dissociated using microwave plasma or hot filament activation technique, thereby providing a reactive environment for promoting diamond growth. In some embodiments, the first layer 808 and the second layer 816 may be formed in a controlled manner to achieve the desired shape and dimensions of the diamond casing 124. In some embodiments, the desired shape and dimension of the diamond casing 124 may be produced by adjusting one or more process parameters, gas composition, pressure, temperature, and growth time. In some embodiments, the method may include doping one or more gases, such as but not limited to, boron, nitrogen, or silicon, into the gas mixture to modify the electrical, thermal, and optical properties of the diamond casing. In some embodiments, the debonding of the first support structure 804 and the second support structure 812 may be performed by a method including, but not limited to, chemical etching, electrochemical etching, laser etching, mechanical polishing, thermal decomposition, or other suitable techniques, depending on the material of the support structure.

In some embodiments, the diamond casing 124 may be fabricated using a 3D sacrificial support structure. The support structure is fabricated to incorporate the geometry of diamond casing 124, including both the first portion 128 and the second portion 132, as well as any necessary features such as receptacles for the electrodes and sensors. The support structure is fabricated using a suitable manufacturing technique, such as 3D printing or advanced machining, and serves as a template for the subsequent diamond growth. The diamond is grown on the support structure using a single CVD process, filling the spaces and cavities to form a seamless, monolithic diamond casing 124. After the diamond growth is complete, the support structure is removed, leaving behind the finished monolithic diamond casing 124. In some embodiments, the electronic device 104, the electrode 120, battery 158, and the sensors may be directly integrated into the support structure. The electronic device 104, the electrode 120, battery 158, and the sensors may be placed in predefined cavities or receptacles within the support structure prior to the diamond growth process. These components are then encapsulated within the monolithic diamond casing 124 during the CVD growth.

With regard to the dome shape of the diamond casing, for example, said shape and geometry of the diamond casing 124 may be obtained by controlling various process parameters during the diamond deposition process. These parameters include, but are not limited to, the substrate design and preparation, plasma enhancement techniques, gas composition and flow rates, temperature and pressure regulation, growth time, and rate control, and post-growth processing steps. By optimizing and regulating these parameters, the diamond growth can be tailored to achieve the desired dome shape, or any other geometry required for the biomedical apparatus 100. As an example, plasma enhancement techniques may be employed to promote preferential diamond growth on the dome-shaped substrate. Additionally, the gas composition, temperature, pressure, and/or growth time, can be adjusted to control the diamond growth rate, thickness, and conformity to the desired shape. Post-growth processing steps, such as laser cutting, selective etching, and polishing, can further refine the dome shape (or any desired shape of the diamond casing 124) and further improve the surface characteristics of the diamond casing 124. Additionally, any excess or undesired growth of diamond on the biomedical apparatus 100 may be removed using known techniques such as laser cutting or etching process.

In some embodiments, the method includes fabricating the diamond casing 124 with a cavity that corresponds to a size and shape of the electronic device 104. After the formation of the diamond casing 124, the method includes inserting the electronic device 104 into the cavity. The method further includes sealing the cavity using a suitable method including, but not limited to, laser welding, brazing, or adhesive bonding, thereby hermetically sealing the electronic device 104 within the diamond casing 124.

In some embodiments, the method includes encapsulating the electronic device 104 within a protective layer or coating of diamond, such as a thin layer of CVD diamond or a biocompatible polymer. The method includes placing the encapsulated electronic device 104 onto the support structure. Thereafter, the method includes growing the diamond casing 124 around the encapsulated electronic device 104 using a CVD process. The method provides an additional layer of protection for the electronic device 104 during the diamond growth process thereby ensuring the compatibility of the electronic device 104 with the diamond casing 124.

In some embodiments, the method includes fabricating the diamond casing 124 in multiple steps, with the electronic device 104 being embedded during an intermediate stage. The method includes fabricating the first portion of the diamond casing 124, such as the base 130, on the support structure using a CVD process. The method further includes placing the electronic device 104 onto the first portion 128 and subsequently fabricating the second portion 132 of the diamond casing 124, such as the dome 134, over the electronic device 104, encapsulating the electronic device 104 within the diamond casing 124.

In some embodiments, the method includes introducing a doping gas such as Boron, Nitrogen, or Hydrogen in the reactor with the mixture of gases. The introduction of the doping gas enhances the electrical and thermal conductivity of the diamond casing 124. For instance, the electrical conductivity of the diamond casing 124 may be enhanced by incorporating dopants, such as boron, during the CVD process. Boron is a commonly used p-type dopant that may significantly increase the electrical conductivity of the diamond casing. The concentration and distribution of boron dopants may be controlled by adjusting the ratio of boron-containing gases (e.g., diborane) to the main carbon-containing gases (e.g., methane) in the CVD gas mixture. The thermal conductivity of the diamond casing 124 is primarily influenced by factors such as grain size, grain orientation, and the presence of defects or impurities. By optimizing the CVD process parameters and minimizing the incorporation of non-diamond carbon phases or impurities, high thermal conductivity may be achieved in the deposited diamond material. In some embodiments, Nitrogen or Phosphorus may also be used to increase the electrical conductivity of the diamond casing 124. Similarly, introducing gases such as Nitrogen, Silicon, or Germanium enhances the thermal conductivity of the diamond casing 124.

The biomedical apparatus 100 may be implantable within a living body such that the encapsulating layer 122 of the electrode 120 transmits electrical signals to the portion of the living body. In some embodiments, the electronic device 104 may include a means for providing stimulation to the portion of the living body. For example, the electronic device 104 may include a cardiac stimulation device configured to deliver therapy in the form of electrical pulses to a cardiac tissue via the encapsulating layer 122 of the electrode 120. Similarly, the electronic device 104 may include a neurostimulation device configured to deliver therapy in the form of electrical pulses to a nerve tissue via the encapsulating layer 122 of the electrode 120.

In some embodiments, the electronic device 104 may include various different types of sensors and/or circuitry configured to detect one or more signals associated with a physiological state within the living body. In some embodiments, the physiological state may include a function and/or a condition of the living body. For example, one or more sensors 178 may be embedded within the dome 134 of the diamond casing 124, thereby allowing the sensor 178 to be in direct contact with a portion of the living body. The sensors 178 may be connected to the electronic device 104 through one or more conductive paths embedded within the diamond casing 124. For example, the sensors 178 may be connected to the electronic device 104 by using one or more metal wires 180 made of material such as gold or platinum, embedded within the diamond casing 124 (see FIG. 3). In some embodiments, the sensors 178 may be connected to the electronic device 104 by one or more conductive channels disposed within the diamond casing 124. The conductive channels may be formed by doping boron thereby increasing the electrical conductivity of the diamond casing 124. In some embodiments, the sensors 178 may be connected to the electronic device 104 by one or more thermal vias (for example the first thermal vias 136). These sensors 178 may include, but are not limited to, sensors, and/or circuitry configured to detect blood pressure, blood flow rate, heart rate, respiratory rate, blood composition, substances within the blood (e.g., oxygen, carbon dioxide or glucose), temperature, activity state (e.g., moving, still, asleep, awake or exercising), speech and/or other physical properties associated with the living body. In some embodiments, the sensors 178 may provide real-time feedback to the electronic device 104 thereby allowing the electronic device 104 to adjust intensity of stimulation to the portion of the living body. In some embodiments, the electronic device 104 may include one or more additional sensors that include, but are not limited to, pressure sensors, blood flow sensors, force sensors, blood composition sensors, optical sensors, accelerometers, piezoelectric sensors, biosensors, acoustic sensors and/or other sensors configured to detect states and/or physical activity of the living body.

In some embodiments, the electronic device may include an array of electrode 120 covered by the encapsulating layer 122 for sensing a plurality of biomarker levels within the living body. For example, the electrode 120 may simultaneously detect biomarker levels related to different injuries, cardiovascular disease, cancer, and infection protein. In some embodiments, the electrode 120 may detect biomarker levels related to nucleic acid from one or more tissue such as, but not limited to microliter sweat, plasma, and interstitial fluid samples without external reagent addition.

In operation, the electronic device 104 produces heat which is transferred to the diamond casing 124 via the first thermal vias 136 and the second thermal vias 142. The diamond casing 124 absorbs the heat from the electronic device 104 and the heat is spread evenly across the diamond casing 124 with the help of the microchannels 144, 150. The microchannels 144, 150 may dissipate heat into the environment, e.g., the portion of the living body, e.g., by way of convection, thereby keeping the biomedical apparatus 100 at an optimal operating temperature.

At the same time, the diamond casing provides electrical conductivity for sending signals to the portion of the living body. For example, the ultrafine tips 164 of the first portion 128 provide electrical signals to the tissue of the living body through the diamond, thereby allowing proper stimulation of the tissue without any distortion in the electrical signal. Hence, diamond improves the electrical conductivity of the biomedical apparatus 100.

Further, the diamond casing 124 also provides optical transparency to the biomedical apparatus 100. For example, the encapsulating layer 122 may include one or more light sources, such as light emitting diodes LEDs, or lasers, configured to provide reliable and focused light-based stimulation of a tissue of the living body. The optical transparency allows precise wavelengths of the light for stimulating and activating a target tissue of the living body, thereby improving light-based treatment methods.

The biomedical apparatus 100 provides a more efficient way of dissipating heat as compared traditional metallic or polymeric casings. A comparative analysis between the performance of the biomedical apparatus 100 versus traditional implants has been provided in Table 1 below:

**Table 1: Comparative analysis between the performance of the biomedical apparatus 100 with diamond casing versus conventional implants of various shapes.**

| S. No | Shape of biomedical apparatus | Type of biomedical apparatus | Peak Temperature | Max Spatial Temperature Variance ΔT | Volume |
|---|---|---|---|---|---|
| 1 | Spherical | Traditional | 45°C | 15°C | 1.2cm3 |
| | | Diamond (100) | 34°C | 8°C | 0.7cm3 |
| 2 | Cylindrical | Traditional | 46°C | 16°C | 1.3cm3 |
| | | Diamond (100) | 38°C | 11°C | 0.8cm3 |
| 3 | Rectangular | Traditional | 47°C | 17°C | 1.4cm3 |
| | | Diamond (100) | 35°C | 9°C | 0.6cm3 |
| 4 | Elliptical | Traditional | 44°C | 14°C | 1.1cm3 |
| | | Diamond (100) | 33°C | 7°C | 0.5cm3 |
| 5 | Capsular | Traditional | 43°C | 13°C | 1.0cm3 |
| | | Diamond (100) | 31°C | 5°C | 0.4cm3 |

The experiment was conducted on an electronic device 104 having a power dissipation of 500 mW and a size of 5 mm × 5 mm × 2 mm. The electronic device 104 consisted of a microcontroller, a wireless communication module, and a sensor array, which are typical components found in implantable medical devices. When a spherical traditional device was used with the electronic device 104, the peak temperature of the electronic device 104 went up to 45°C, while the Max Spatial temperature variance (ΔT) went up to 15 °C while the volume of the conventional implants that were used in the experiment was 1.2 cm3. The term "peak temperature" may be defined as the highest temperature attained by the biomedical apparatus 100 during operation. The term "volume" may be defined as a three-dimensional space taken by the biomedical apparatus 100 inside the living body. The term "Max Spatial temperature variance" may be defined as the difference of temperature at a particular location at different time. However, when a spherical diamond casing 124 was used with the electronic device 104, the peak temperature of the electronic device 104 went up to 34°C, while the Max Spatial temperature variance (ΔT) went up to 8 °C while the volume of the conventional implants that were used in the experiment was 0.7 cm3.

Further, when a cylindrical traditional device was used with the electronic device 104, the peak temperature of the electronic device 104 went up to 46°C, while the Max Spatial temperature variance (ΔT) went up to 16 °C while the volume of the conventional implants that were used in the experiment was 1.3 cm3. On the contrary, when the cylindrical diamond casing 124 was used with the same electronic device 104, the peak temperature of the electronic device 104 went only up to 38°C, while ΔT was 11 °C while the volume of the cylindrical diamond casing that was used in the experiment was 0.8 cm3.

Further, when a rectangular traditional device was used with the electronic device 104, the peak temperature of the electronic device 104 went up to 47°C, while the Max Spatial temperature variance (ΔT) went up to 17 °C while the volume of the conventional implants that were used in the experiment was 1.4 cm3. However, when a rectangular diamond casing 124 was used with the same electronic device 104, the peak temperature of the electronic device 104 went up only up to 35°C, while ΔT went up to 9°C while the volume of the rectangular diamond casing was only 0.6 cm3.

Similarly, when an elliptical traditional device was used with the electronic device 104, the peak temperature of the electronic device 104 went up to 44°C, while the Max Spatial temperature variance (ΔT) went up to 14 °C while the volume of the conventional implants that were used in the experiment was 1.1 cm3. However, when an elliptical diamond casing 124 was used with the same electronic device 104, the peak temperature of the electronic device 104 went up to 33°C, while ΔT went up to 7°C while the volume of the elliptical diamond casing was only 0.5 cm3.

Further, when a capsular traditional device was used with the electronic device 104, the peak temperature of the electronic device 104 went up to 43°C, while the Max Spatial temperature variance (ΔT) went up to 13 °C while the volume of the conventional implants that were used in the experiment was 1.0 cm3. On the other hand, when a Capsular diamond casing was used with the same electronic device 104, the peak temperature of the electronic device 104 went up to 31°C, while ΔT went up to 5°C while the volume of the Capsular diamond casing was only 0.4 cm3. Hence, it is very evident that diamond casing with any shape had the better heat dissipating efficiency and the smallest size. This helps keep the biomedical apparatus 100 and provides up to ninety percent volume saving. The capsular diamond casing had the lowest peak temperatures and thermal gradients across a biomedical apparatus 100 indicating the most efficient heat distribution.

Exemplary applications of the biomedical apparatus 100 of the present disclosure will now be explained in detail. To this end, the biomedical apparatus 100 may be used in cardiac pacemakers and resynchronization devices, neural stimulators and neurostimulation devices, cochlear implants, retinal prosthesis, muscular stimulators, electroacupuncture devices, implanted analyzers and sensors, other implantable electrostimulation devices.

The biomedical apparatus 100 may be used in a drug delivery system adapted to incorporate drug reservoirs and release mechanisms, enabling targeted and controlled delivery of therapeutic agents within the living body. The diamond casing 124 provides a chemically inert and biocompatible interface, while the electronic device 104 can control the timing and dosage of drug release.

The biomedical apparatus 100 may be used in neuroscience research tools. The biomedical apparatus 100 may be used to investigate brain function, study neurological disorders, and develop neuromodulation therapies. The diamond casing 124 provides a stable and biocompatible interface for long-term neural implantation.

The biomedical apparatus 100 may be used in regenerative medicine and tissue engineering: The biomedical apparatus 100 may be integrated with scaffolds and biomaterials to create smart implants for tissue regeneration. The device can provide electrical and optical stimulation to guide cell growth and differentiation, while also monitoring the physiological microenvironment to optimize the regenerative process.

## Claims

1. A biomedical apparatus (100) for implantation within a living body, the biomedical apparatus comprising:
an electrode (120) including one or more tips (154);
an electronic device (104) configured to transmit electrical signals to a portion of the living body through the electrode (120); and
a monolithic diamond casing (124) for housing and hermetically sealing the electronic device (104) and the electrode (120) therewithin, wherein the monolithic diamond casing (124) includes:
a first portion (128) defining an encapsulating layer (122) around the one or more tips (154) of the electrode (120), the first portion (128) configured to define an electrical path between the electrode (120) and the portion of the living body; and
a second portion (132) enwrapping the electronic device (104) and the electrode (120) and being integral and seamlessly contiguous to the first portion (128), the second portion (132) defining one or more heat dissipation structures (126) to dissipate heat generated from the electronic device (104) away from the monolithic diamond casing (124).

2. The biomedical apparatus (100) as claimed in claim 1, wherein the electrode (120) is an integral part of the monolithic diamond casing (124) and is formed from the same material as that of the monolithic diamond casing (124).

3. The biomedical apparatus (100) as claimed in claim 1, wherein the first portion (128) of the monolithic diamond casing (124) includes a base (130) for coupling and mounting the electronic device (104) and the electrode (120) thereto and includes one or more first thermal vias (136) extending from the base (130), the one or more thermal vias (136) ingressing, at least partly, into the electronic device (104) and the electrode (120) for transferring heat from the electronic device (104) and the electrode (120) to the base (130).

4. The biomedical apparatus (100) as claimed in claim 3, wherein the second portion (132) outwardly projects from the first portion (128) and wherein the second portion (132) of the monolithic diamond casing (124) includes a dome (134) at least partly surrounding the electronic device (104), the dome (134) defining a clearance (C) within the monolithic diamond casing (124) with respect to the base (130) and the electronic device (104).

5. The biomedical apparatus (100) as claimed in claim 4, wherein the one or more heat dissipation structures (126) include one or more second thermal vias (142) extending from the dome (134) and being in contact with or ingressing at least party into the electronic device (104).

6. The biomedical apparatus (100) as claimed in claim 1, wherein the one or more heat dissipation structures (126) include one or more microchannels (144, 150).

7. The biomedical apparatus (100) as claimed in claim 6, wherein the one or more microchannels (144,150) include an irregular and/or a nonlinear profile, and the one or more microchannels, independently or combinedly define, one or more of a fractal profile, a sinusoidal profile, a grid profile, or any combination thereof.

8. The biomedical apparatus (100) as claimed in claim 1, wherein the second portion (132) includes a varying cross-sectional area across an expanse of the second portion (132), wherein regions of the second portion (132) having greater cross-sectional area dissipate relatively more heat than the regions of the second portion (132) having lower cross-sectional area.

9. The biomedical apparatus (100) as claimed in claim 1, wherein the monolithic diamond casing (124) includes a non-uniform grain size distribution and a varying grain orientation, wherein regions of the monolithic diamond casing (124) having lower grain size distribution provides relatively more thermal conductivity than the regions of the monolithic diamond casing (124) having higher grain size distribution.

10. The biomedical apparatus (100) as claimed in claim 1, wherein the first portion (128) includes one or more receptacles (162) to correspondingly receive the one or more tips (154) of the electrode (120), the one or more receptacles (162) defining ultrafine tips (164) having diameter ranging from 0.1 µm to 5 µm and lengths of 10 µm to 100 µm.

11. The biomedical apparatus (100) as claimed in claim 1, wherein the monolithic diamond casing (124) is produced by an additive manufacturing process or a chemical vapor deposition (CVD) process.

12. The biomedical apparatus (100) as claimed in claim 1, wherein the monolithic diamond casing (124) is produced by doping diamond with Boron, Hydrogen and/or Nitrogen.

13. The biomedical apparatus (100) as claimed in claim 1, wherein a thermal interface material or microfluid is disposed between the first portion (128) and the second portion (132).

14. The biomedical apparatus (100) as claimed in claim 1, wherein vacuum is present between the first portion (128) and the second portion (132).

15. The biomedical apparatus (100) as claimed in claim 1, wherein the monolithic diamond casing (124) is produced from a single crystalline or polycrystalline diamond.

16. The biomedical apparatus (100) as claimed in claim 1, wherein the monolithic diamond casing (124) includes one or more sensors (178) embedded therein to be in direct contact with the portion of the living body.

17. The biomedical apparatus (100) as claimed in claim 1, wherein the electronic device (104) includes a battery (158) for supplying power to the electronic device (104), and the electrode (120).

18. The biomedical apparatus (100) as claimed in claim 1, wherein the electronic device (104) includes an array of electrodes (120) for sensing a plurality of biomarker levels within the living body.

19. A method for producing a biomedical apparatus (100), the method comprises:
providing a first support structure (804) including a first seed layer of diamond inside a chemical vapor deposition (CVD) reactor, wherein the first support structure (804) is one of a mold or a template;
forming a first layer (808) of diamond on the first seed layer to form a first portion (128) of a monolithic diamond casing (124);
debonding the first portion (128) of the monolithic diamond casing (124) from the first support structure (804);
providing a second support structure (812) including a second seed layer of diamond inside the CVD reactor, wherein the second support structure (812) is one of a mold or a template;
forming a second layer (816) of diamond on the second seed layer to form a second portion (132) of a monolithic diamond casing (124);
debonding the second portion (132) of the monolithic diamond casing (124) from the second support structure (812);
positioning an electrode (120) on the first portion (128) such that the first portion (128) defines an encapsulating layer (122) around one or more tips (154) of the electrode (120), the first portion (128) configured to define an electrical path between the electrode (120) and a portion of a living body;
providing an electronic device (104) on the first portion (128) to transmit electrical signals to a portion of the living body through the electrode (120); and
joining the first portion (128) and the second portion (132) to form the monolithic diamond casing (124) such that the second portion (132) enwraps the electronic device (104) and is integral and seamlessly contiguous to the first portion (128), the second portion (132) defining one or more heat dissipation structures (126) to dissipate heat generated from the electronic device (104) away from the monolithic diamond casing (124).

20. The method of claim 19, wherein the first layer (808) and the second layer (816) of diamond are formed by filling the CVD reactor with a mixture of gases and ionizing the mixture of gases to provide a reactive environment for promoting diamond growth, and wherein the mixture of gases is doped with at least one of Boron, Hydrogen and/or Nitrogen.

## Patentansprüche

1. Biomedizinische Vorrichtung (100) zur Implantation in einen lebenden Körper, wobei die biomedizinische Vorrichtung umfasst:
eine Elektrode (120) mit einer oder mehreren Spitzen (154);
eine elektronische Vorrichtung (104), die so konfiguriert ist, dass sie elektrische Signale über die Elektrode (120) an einen Bereich des lebenden Körpers überträgt; und
ein monolithisches Diamantgehäuse (124) zur Aufnahme und hermetischen Abdichtung der elektronischen Vorrichtung (104) und der Elektrode (120) darin, wobei das monolithische Diamantgehäuse (124) umfasst:
den ersten Teil (128), der eine Verkapselungsschicht (122) um die eine oder mehrere Spitzen (154) der Elektrode (120) herum bildet, wobei der erste Teil (128) so ausgebildet ist, dass er einen elektrischen Pfad zwischen der Elektrode (120) und dem Bereich des lebenden Körpers bildet; und
den zweiten Teil (132), der die elektronische Vorrichtung (104) und die Elektrode (120) umhüllt und einstückig und nahtlos an den ersten Teil (128) angrenzt, wobei der zweite Teil (132) eine oder mehrere Wärmeableitungsstrukturen (126) definiert, um von der elektronischen Vorrichtung (104) erzeugte Wärme vom monolithischen Diamantgehäuse (124) abzuleiten.

2. Biomedizinische Vorrichtung (100) nach dem Anspruch 1, wobei die Elektrode (120) ein integraler Bestandteil des monolithischen Diamantgehäuses (124) ist und aus demselben Material wie das monolithische Diamantgehäuse (124) besteht.

3. Biomedizinische Vorrichtung (100) nach dem Anspruch 1, wobei der erste Teil (128) des monolithischen Diamantgehäuses (124) eine Basis (130) zum Verbinden und Befestigen der elektronischen Vorrichtung (104) und der Elektrode (120) und eine oder mehrere erste thermische Durchkontaktierungen (136) aufweist, die sich von der Basis (130) aus erstrecken, wobei die eine oder mehreren thermischen Durchkontaktierungen (136) zumindest teilweise in die elektronische Vorrichtung (104) und die Elektrode (120) hineinragen, um Wärme von der elektronischen Vorrichtung (104) und der Elektrode (120) an die Basis (130) zu übertragen.

4. Biomedizinische Vorrichtung (100) nach dem Anspruch 3, wobei der zweite Teil (132) vom ersten Teil (128) nach außen vorsteht und wobei der zweite Teil (132) des monolithischen Diamantgehäuses (124) eine Kuppel (134) aufweist, die die elektronische Vorrichtung (104) zumindest teilweise umgibt, wobei die Kuppel (134) innerhalb des monolithischen Diamantgehäuses (124) einen Freiraum (C) in Bezug auf den Sockel (130) und die elektronische Vorrichtung (104) definiert.

5. Biomedizinische Vorrichtung (100) nach dem Anspruch 4, wobei die eine oder die mehreren Wärmeableitungsstrukturen (126) eine oder mehrere zweite thermische Durchkontaktierungen (142) umfassen, die sich von der Kuppel (134) aus erstrecken und mit der elektronischen Vorrichtung (104) in Kontakt stehen oder zumindest teilweise in diese hineinragen.

6. Biomedizinische Vorrichtung (100) nach dem Anspruch 1, wobei die eine oder die mehreren Wärmeableitungsstrukturen (126) einen oder mehrere Mikrokanäle (144, 150) umfassen.

7. Biomedizinische Vorrichtung (100) nach dem Anspruch 6, wobei der eine oder die mehreren Mikrokanäle (144, 150) ein unregelmäßiges und/oder ein nichtlineares Profil aufweisen und der eine oder die mehreren Mikrokanäle - unabhängig voneinander oder in Kombination - eines oder mehrere der folgenden Profile definieren: ein fraktales Profil, ein sinusförmiges Profil, ein Gitterprofil oder eine beliebige Kombination davon.

8. Biomedizinische Vorrichtung (100) nach dem Anspruch 1, wobei der zweite Teil (132) über seine gesamte Ausdehnung eine sich ändernde Querschnittsfläche aufweist, wobei die Bereiche des zweiten Teils (132) mit größerer Querschnittsfläche relativ mehr Wärme abgeben als die Bereiche des zweiten Teils (132) mit geringerer Querschnittsfläche.

9. Biomedizinische Vorrichtung (100) nach dem Anspruch 1, wobei das monolithische Diamantgehäuse (124) eine ungleichmäßige Korngrößenverteilung und eine variierende Kornorientierung aufweist, wobei Bereiche des monolithischen Diamantgehäuses (124) mit einer geringeren Korngrößenverteilung eine relativ höhere Wärmeleitfähigkeit aufweisen als die Bereiche des monolithischen Diamantgehäuses (124) mit einer höheren Korngrößenverteilung.

10. Biomedizinische Vorrichtung (100) nach dem Anspruch 1, wobei der erste Abschnitt (128) eine oder mehrere Aufnahmen (162) umfasst, um die eine oder mehreren Spitzen (154) der Elektrode (120) entsprechend aufzunehmen, wobei die eine oder mehreren Aufnahmen (162) ultrafeine Spitzen (164) definieren, deren Durchmesser im Bereich von 0,1 pm bis 5 pm und deren Länge im Bereich von 10 pm bis 100 pm liegt.

11. Biomedizinische Vorrichtung (100) nach dem Anspruch 1, wobei das monolithische Diamantgehäuse (124) durch ein additives Fertigungsverfahren oder ein chemisches Gasphasenabscheidungsverfahren (CVD) hergestellt wird.

12. Biomedizinische Vorrichtung (100) nach dem Anspruch 1, wobei das monolithische Diamantgehäuse (124) durch Dotierung von Diamant mit Bor, Wasserstoff und/oder Stickstoff hergestellt wird.

13. Biomedizinische Vorrichtung (100) nach dem Anspruch 1, wobei zwischen dem ersten Abschnitt (128) und dem zweiten Abschnitt (132) ein Wärmeleitmaterial oder ein Mikrofluid angeordnet ist.

14. Biomedizinische Vorrichtung (100) nach dem Anspruch 1, wobei zwischen dem ersten Teil (128) und dem zweiten Teil (132) eine Vakuumzone vorhanden ist.

15. Die biomedizinische Vorrichtung (100) nach dem Anspruch 1, wobei das monolithische Diamantgehäuse (124) aus einem einkristallinen oder polykristallinen Diamanten hergestellt ist.

16. Biomedizinische Vorrichtung (100) nach dem Anspruch 1, wobei das monolithische Diamantgehäuse (124) einen oder mehrere darin eingebettete Sensoren (178) umfasst, die in direktem Kontakt mit dem Teil des lebenden Körpers stehen.

17. Biomedizinische Vorrichtung (100) nach dem Anspruch 1, wobei die elektronische Einrichtung (104) eine Batterie (158) zur Stromversorgung der elektronischen Einrichtung (104) und der Elektrode (120) umfasst.

18. Biomedizinische Vorrichtung (100) nach dem Anspruch 1, wobei die elektronische Einrichtung (104) eine Anordnung von Elektroden (120) zum Erfassen einer Vielzahl von Biomarker-Konzentrationen im lebenden Körper umfasst.

19. Das Verfahren zur Herstellung einer biomedizinischen Vorrichtung (100), wobei das Verfahren umfasst:
das Bereitstellen der ersten Trägerstruktur (804) mit der ersten Keimschicht aus Diamant innerhalb eines CVD-Reaktors (Chemical Vapor Deposition), wobei die erste Trägerstruktur (804) entweder eine Form oder eine Schablone ist;
das Bilden der ersten Diamantschicht (808) auf der ersten Keimschicht, um den ersten Teil (128) eines monolithischen Diamantgehäuses (124) zu bilden;
das Ablösen des ersten Teils (128) des monolithischen Diamantgehäuses (124) von der ersten Trägerstruktur (804);
das Bereitstellen der zweiten Trägerstruktur (812) mit der zweiten Keimschicht aus Diamant im Inneren des CVD-Reaktors, wobei die zweite Trägerstruktur (812) entweder eine Form oder eine Schablone ist;
das Bilden der zweiten Schicht (816) aus Diamant auf der zweiten Keimschicht, um den zweiten Teil (132) eines monolithischen Diamantgehäuses (124) zu bilden;
das Ablösen des zweiten Teils (132) des monolithischen Diamantgehäuses (124) von der zweiten Stützstruktur (812);
das Positionieren einer Elektrode (120) auf dem ersten Teil (128) derart, dass der erste Teil (128) eine umhüllende Schicht (122) um eine oder mehrere Spitzen (154) der Elektrode (120) bildet, wobei der erste Teil (128) so ausgebildet ist, dass er einen elektrischen Pfad zwischen der Elektrode (120) und einem Teil eines lebenden Körpers bildet;
das Bereitstellen einer elektronischen Vorrichtung (104) auf dem ersten Teil (128), um elektrische Signale über die Elektrode (120) an einen Teil des lebenden Körpers zu übertragen; und
das Verbinden des ersten Teils (128) und des zweiten Teils (132) zur Bildung des monolithischen Diamantgehäuses (124), derart, dass der zweite Teil (132) die elektronische Vorrichtung (104) umhüllt und einstückig und nahtlos an den ersten Teil (128) angrenzt, wobei der zweite Abschnitt (132) eine oder mehrere Wärmeableitungsstrukturen (126) definiert, um die von der elektronischen Vorrichtung (104) erzeugte Wärme vom monolithischen Diamantgehäuse (124) weg abzuleiten.

20. Das Verfahren nach dem Anspruch 19, wobei die erste Schicht (808) und die zweite Schicht (816) aus Diamant gebildet werden, indem der CVD-Reaktor mit einem Gasgemisch befüllt und das Gasgemisch ionisiert wird, um eine reaktive Umgebung zur Förderung des Diamantwachstums zu schaffen, und wobei das Gasgemisch mit mindestens einem der Stoffe Bor, Wasserstoff und/oder Stickstoff dotiert ist.

## Revendications

1. Un appareil biomédical (100) destiné à être implanté dans un corps vivant, l'appareil biomédical comprenant :
une électrode (120) comprenant une ou plusieurs pointes (154) ;
un dispositif électronique (104) configuré pour transmettre des signaux électriques à une partie du corps vivant par l'intermédiaire de l'électrode (120) ; et
un boîtier monolithique en diamant (124) destiné à loger et à assurer l'étanchéité hermétique du dispositif électronique (104) et de l'électrode (120) à l'intérieur de celui-ci, dans lequel le boîtier monolithique en diamant (124) comprend :
une première partie (128) définissant une couche d'encapsulation (122) autour de la ou des pointes (154) de l'électrode (120), la première partie (128) étant configurée pour définir un trajet électrique entre l'électrode (120) et la partie du corps vivant ; et
une deuxième partie (132) enveloppant le dispositif électronique (104) et l'électrode (120) et étant intégrale et contiguë sans joint à la première partie (128), la deuxième partie (132) définissant une ou plusieurs structures de dissipation thermique (126) pour dissiper la chaleur générée par le dispositif électronique (104) à l'écart du boîtier monolithique en diamant (124).

2. L'appareil biomédical (100) selon la revendication 1, dans lequel l'électrode (120) est une partie intégrale du boîtier monolithique en diamant (124) et est formée du même matériau que celui du boîtier monolithique en diamant (124).

3. L'appareil biomédical (100) selon la revendication 1, dans lequel la première partie (128) du boîtier monolithique en diamant (124) comprend une base (130) destinée à coupler et monter le dispositif électronique (104) et l'électrode (120) sur celle-ci et comprend une ou plusieurs premières traversées thermiques (136) s'étendant à partir de la base (130), la ou les traversées thermiques (136) pénétrant, au moins en partie, dans le dispositif électronique (104) et l'électrode (120) pour transférer la chaleur du dispositif électronique (104) et de l'électrode (120) à la base (130).

4. L'appareil biomédical (100) selon la revendication 3, dans lequel la deuxième partie (132) fait saillie vers l'extérieur à partir de la première partie (128) et dans lequel la deuxième partie (132) du boîtier monolithique en diamant (124) comprend un dôme (134) entourant au moins en partie le dispositif électronique (104), le dôme (134) définissant un espace libre (C) à l'intérieur du boîtier monolithique en diamant (124) par rapport à la base (130) et au dispositif électronique (104).

5. L'appareil biomédical (100) selon la revendication 4, dans lequel la ou les structures de dissipation thermique (126) comprennent une ou plusieurs deuxièmes traversées thermiques (142) s'étendant à partir du dôme (134) et étant en contact avec ou pénétrant au moins en partie dans le dispositif électronique (104).

6. L'appareil biomédical (100) selon la revendication 1, dans lequel la ou les structures de dissipation thermique (126) comprennent un ou plusieurs microcanaux (144, 150).

7. L'appareil biomédical (100) selon la revendication 6, dans lequel le ou les microcanaux (144,150) comprennent un profil irrégulier et/ou non linéaire, et le ou les microcanaux, individuellement ou conjointement, définissent un ou plusieurs parmi un profil fractal, un profil sinusoïdal, un profil en grille, ou toute combinaison de ceux-ci.

8. L'appareil biomédical (100) selon la revendication 1, dans lequel la deuxième partie (132) comprend une aire de section transversale variable sur une étendue de la deuxième partie (132), dans lequel des régions de la deuxième partie (132) ayant une aire de section transversale plus grande dissipent relativement plus de chaleur que les régions de la deuxième partie (132) ayant une aire de section transversale plus faible.

9. L'appareil biomédical (100) selon la revendication 1, dans lequel le boîtier monolithique en diamant (124) comprend une distribution de taille de grains non uniforme et une orientation de grains variable, dans lequel des régions du boîtier monolithique en diamant (124) ayant une distribution de taille de grains plus faible fournissent une conductivité thermique relativement plus élevée que les régions du boîtier monolithique en diamant (124) ayant une distribution de taille de grains plus élevée.

10. L'appareil biomédical (100) selon la revendication 1, dans lequel la première partie (128) comprend un ou plusieurs réceptacles (162) destinés à recevoir de manière correspondante la ou les pointes (154) de l'électrode (120), le ou les réceptacles (162) définissant des pointes ultrafines (164) ayant un diamètre allant de 0,1 µm à 5 µm et des longueurs de 10 µm à 100 µm.

11. L'appareil biomédical (100) selon la revendication 1, dans lequel le boîtier monolithique en diamant (124) est produit par un procédé de fabrication additive ou un procédé de dépôt chimique en phase vapeur (CVD).

12. L'appareil biomédical (100) selon la revendication 1, dans lequel le boîtier monolithique en diamant (124) est produit par dopage du diamant avec du Bore, de l'Hydrogène et/ou de l'Azote.

13. L'appareil biomédical (100) selon la revendication 1, dans lequel un matériau d'interface thermique ou un microfluide est disposé entre la première partie (128) et la deuxième partie (132).

14. L'appareil biomédical (100) selon la revendication 1, dans lequel du vide est présent entre la première partie (128) et la deuxième partie (132).

15. L'appareil biomédical (100) selon la revendication 1, dans lequel le boîtier monolithique en diamant (124) est produit à partir d'un diamant monocristallin ou polycristallin.

16. L'appareil biomédical (100) selon la revendication 1, dans lequel le boîtier monolithique en diamant (124) comprend un ou plusieurs capteurs (178) intégrés dans celui-ci pour être en contact direct avec la partie du corps vivant.

17. L'appareil biomédical (100) selon la revendication 1, dans lequel le dispositif électronique (104) comprend une batterie (158) destinée à fournir de l'énergie au dispositif électronique (104), et à l'électrode (120).

18. L'appareil biomédical (100) selon la revendication 1, dans lequel le dispositif électronique (104) comprend un réseau d'électrodes (120) destiné à détecter une pluralité de niveaux de biomarqueurs dans le corps vivant.

19. Procédé de production d'un appareil biomédical (100), le procédé comprend :
fournir une première structure de support (804) comprenant une première couche germe de diamant à l'intérieur d'un réacteur de dépôt chimique en phase vapeur (CVD), dans lequel la première structure de support (804) est l'un d'un moule ou d'un gabarit ;
former une première couche (808) de diamant sur la première couche germe pour former une première partie (128) d'un boîtier monolithique en diamant (124) ;
désolidariser la première partie (128) du boîtier monolithique en diamant (124) de la première structure de support (804) ;
fournir une deuxième structure de support (812) comprenant une deuxième couche germe de diamant à l'intérieur du réacteur CVD, dans lequel la deuxième structure de support (812) est l'un d'un moule ou d'un gabarit ;
former une deuxième couche (816) de diamant sur la deuxième couche germe pour former une deuxième partie (132) d'un boîtier monolithique en diamant (124) ;
désolidariser la deuxième partie (132) du boîtier monolithique en diamant (124) de la deuxième structure de support (812) ;
positionner une électrode (120) sur la première partie (128) de sorte que la première partie (128) définisse une couche d'encapsulation (122) autour d'une ou plusieurs pointes (154) de l'électrode (120), la première partie (128) étant configurée pour définir un trajet électrique entre l'électrode (120) et une partie d'un corps vivant ;
fournir un dispositif électronique (104) sur la première partie (128) pour transmettre des signaux électriques à une partie du corps vivant par l'intermédiaire de l'électrode (120) ; et
joindre la première partie (128) et la deuxième partie (132) pour former le boîtier monolithique en diamant (124) de sorte que la deuxième partie (132) enveloppe le dispositif électronique (104) et soit intégrale et contiguë sans joint à la première partie (128), la deuxième partie (132) définissant une ou plusieurs structures de dissipation thermique (126) pour dissiper la chaleur générée par le dispositif électronique (104) à l'écart du boîtier monolithique en diamant (124).

20. Procédé selon la revendication 19, dans lequel la première couche (808) et la deuxième couche (816) de diamant sont formées en remplissant le réacteur CVD avec un mélange de gaz et en ionisant le mélange de gaz pour fournir un environnement réactif destiné à favoriser la croissance du diamant, et dans lequel le mélange de gaz est dopé avec au moins l'un parmi le Bore, l'Hydrogène et/ou l'Azote.
